# EUROPEAN PATENT SPECIFICATION

(11) **EP 1 546 194 B1**
(45) Date of publication and mention of the grant of the patent: **14.03.2012**
(21) Application number: 03756554.6
(22) Date of filing: 01.10.2003
(51) Int. Cl.: C07K 14/195

(54) **THERAPEUTIC USPA1-DERIVED PEPTIDES**
THERAPEUTISCHE, SICH VON USPA1 ABLEITENDE PEPTIDE
PEPTIDES THERAPEUTIQUES DERIVES D'USPA1

(30) Priority: 02.10.2002 GB 0222764
(43) Date of publication of application: 29.06.2005
(60) Divisional application: 10177682.1
(73) Proprietor: UNIVERSITY OF BRISTOL, Clifton Bristol BS8 1TH (GB)
(72) Inventor: VIRJI, M. Department of Pathology and Microbiology, Bristol BS8 1TD (GB)
(74) Representative: McQueen, Andrew Peter
(86) International application number: PCT/GB2003/004273
(87) International publication number: WO 2004/031236

(56) References cited:
- WO-A-98/28333
- DATABASE EMBL [Online] Sequence of Uspa1, 1 November 1999 (1999-11-01) retrieved from EBI Database accession no. Q9XD56 XP002273230
- HILL DARRYL J ET AL: "A novel cell-binding mechanism of Moraxella catarrhalis ubiquitous surface protein UspA: Specific targeting of the N-domain of carcinoembryonic antigen-related cell adhesion molecules by UspA1." MOLECULAR MICROBIOLOGY, vol. 48, no. 1, April 2003 (2003-04), pages 117-129, XP002273226 ISSN: 0950-382X (ISSN print)
- LAFONTAINE ERIC R ET AL: "The UspA1 protein and a second type of UspA2 protein mediate adherence of Moraxella catarrhalis to human epithelial cells in vitro" JOURNAL OF BACTERIOLOGY, vol. 182, no. 5, March 2000 (2000-03), pages 1364-1373, XP002273227 ISSN: 0021-9193
- MEIER P S ET AL: "The outer membrane proteins UspA1 and UspA2 of Moraxella catarrhalis are highly conserved in nasopharyngeal isolates from young children" VACCINE, BUTTERWORTH SCIENTIFIC. GUILDFORD, GB, vol. 20, no. 13-14, 15 March 2002 (2002-03-15), pages 1754-1760, XP004366011 ISSN: 0264-410X
- CHEN ET AL: "Evaluation of purified UspA from Moraxella catarrhalis as a vaccine in a murine model after active immunization" INFECTION AND IMMUNITY, AMERICAN SOCIETY FOR MICROBIOLOGY. WASHINGTON, US, vol. 64, no. 6, 1 June 1996 (1996-06-01), pages 1900-1905, XP002079321 ISSN: 0019-9567
- VIRJI MUMTAZ ET AL: "Carcinoembryonic antigens are targeted by diverse strains of typable and non-typable Haemophilus influenzae" MOLECULAR MICROBIOLOGY, vol. 36, no. 4, May 2000 (2000-05), pages 784-795, XP002273228 ISSN: 0950-382X
- VIRJI MUMTAZ ET AL: "Critical determinants of host receptor targeting by Neisseria meningitidis and Neisseria gonorrhoeae: Identification of Opa adhesiotopes on the N-domain of CD66 molecules" MOLECULAR MICROBIOLOGY, vol. 34, no. 3, November 1999 (1999-11), pages 538-551, XP002273229 ISSN: 0950-382X

## Description

This disclosure relates to therapeutic peptides and in particular to therapeutic peptides which are useful in the preparation of vaccines or other treatments for infection as well as in the screening of compounds for potential pharmaceutical activity in the treatment of infections.

The present inventors have previously identified that Carcinoembryonic antigen related cell adhesion molecules (CEACAMs) are receptors for pathogens of mucosal membranes, especially for respiratory pathogens such as *Neisseria meningitidis, Haemophilus influenzae* and *Moraxella catarrhalis.* CEACAMs belong to the CarcinoEmbryonic Antigen (CEA) family, a member of the Immunoglobulin superfamily. The CEA gene family comprises surface expressed (CEA) and secreted (pregnancy-specific glycoprotein, PSG) subfamilies. The membrane-associated sub-family redefined as CEACAM (CEA-related cell adhesion molecule) ²⁰ comprises several related glycoproteins of which CEACAM1 is the most widely expressed in distinct human tissues ¹². The studies reported by the inventors primarily used Chinese Hamster Ovary (CHO) cells transfected with CEACAM1 (previously termed CD66a and BGPc) that contains four extracellular domains, a TM region and a short (S) or a long (L) cytoplasmic tail (molecular formula: NA1BA2-TM-S or L). In addition, soluble truncated constructs containing one or more of the extracellular domains were used. Previous studies demonstrated that both *Neisseria meningitidis* and *Haemophilus influenzae* primarily target the N-domain of several CEACAMs ^{7,9,10}. Such targeting may lead to cell surface attachment as well as cellular invasion ⁹. In addition, bacteria may bind to CEACAMs on phagocytic cells and T and B lymphocytes. Such interactions may lead to bacterial cell death ⁸, target cell death or inhibition of immune function, e.g. of T and B lymphcytes when *N. gonorrhoeae* (closely related to *N. meningitidis)* binds to CEACAMs of these lymphocytes ^{21,22}. WO 98/28333 describes the sequence of UspA1 from *M*. *catarrhalis* strain O35E (UspA1 O35E and identifies the epitope which binds to antibody (MAb 17C7). Chen et al (1996) Infection and Immunity, 64, 6, 1900-1904 shows that UspA1 from *M. catarrhalis* strain O35E is immunogenic in mice and suggests its use in a vaccine.

The presence of CEACAM-binding ligands in *Moraxella catarrhalis* and *Haemophilus influenzae* has been a surprising find to the present inventors since CEACAMs have long been associated with outer membrane opacity-associated Opa proteins of *Neisseriae* and neither *Haemophilus influenzae* nor *Moraxella catarrhalis* produce Opa proteins. In the description which follows, the present invention will be described with particular reference to infection of the mucosal membranes, especially of the respiratory membranes, or to infection of the ear (especially otitis media) but it is to be understood that the invention finds equal utility in other areas such as the genital mucosa or the urethrae where CEACAM receptors are implicated in infection or other receptor-binding processes or elsewhere in human infection where bacteria may become disseminated from mucosal surfaces.

The mucosal pathogens *Neisseria meningitidis* (Nm), *Haemophilus influenzae* (Hi) and *Moraxella catarrhalis* (Mx) are human specific organisms and reside in the upper respiratory tract from where they may disseminate to cause serious infections. Meningococcal strains of distinct serogroups may be carried within the nasopharynx of up to 25% of healthy individuals¹. However, in a number of subjects, the organism invades the mucosal barrier to cause one of the most rapidly advancing and extremely serious diseases. The precise factors that increase host susceptibility to meningococcal infection are not fully understood. Moreover, the limited protection afforded by group-specific vaccines and the non-immunogenicity of the group B polysaccharide underscore the need for fundamental studies to understand host susceptibility and identify salient sub-capsular features that could serve as common targets to combat meningococci. Studies by the present inventors have provided an understanding of the molecular basis of meningococcal colonisation, the nature of its interactions with human barrier cells (epithelial and endothelial) as well as phagocytic cells. In recent years, the basis for mucosal colonisation by commensal *Neisseriae* has been investigated to understand the features which differentiate between largely harmless colonisers and occasional but serious pathogens such as Nm. In addition, the studies have determined whether commensal *Neisseriae* could be used as carriers of potential vaccine antigens of Nm.

Up to 75% of healthy individuals may carry strains belonging to the species *H*. *influenzae* ². Although as a result of the Hib vaccine, there has been a dramatic decrease in the incidence of the type b disease in the West, diseases caused by non-typable Hi (NTHi) strains remain a major problem. NTHi cause localised as well as disseminated infections including epiglottitis, otitis media, cellulitis, pneumonia, endocarditis, bacteraemia and meningitis. Otitis media is one of the major problems in paediatric medicine and NTHi are responsible for over 20% of episodes in children during the first year of life ^{2,3}. NTHi are also associated with acute recurrent and persistent infections in patients with chronic obstructive pulmonary disease (COPD) and cystic fibrosis. What determines recurrent infections by NTHi in these patients or multiple episodes of otitis media in children is unclear ^{2,3,4}

*Moraxella catarrhalis,* another resident of the human respiratory tract, is often isolated from cases of localised infections together with Hi. Both organisms are associated with sinusitis and exacerbations of asthmatic conditions ^{5,6}. Mx is the third most common cause of otitis media in children (estimated to be responsible for 3-4 million cases annually). It also causes lower respiratory tract infections in adults especially in patients with COPD ⁵. On rare occasions, it has been associated with disseminated infections ⁵. Both Hi and Mx cause persistent infections and are believed to escape host immune mechanisms and antibiotics by tissue penetration ⁴. Several outer membrane proteins of Mx have been studied with respect to their adhesive properties. However, few cellular receptors for Mx have been identified and many of the details of pathogenic mechanisms remain to be investigated ^{4,5,6}.

A primary requirement for respiratory mucosal pathogens is establishment of firm contact with respiratory epithelial cells. The targets of these human tropic pathogens are human specific molecules and studies have to rely on *in vitro* human tissue and organ cultures. The attachment is often mediated by bacterial phase- and antigenically-variable structures. In addition, it is becoming increasingly clear that attachment of pathogens is multi-faceted and environmental adaptation plays a significant role in the manner of attachment. Although many recent studies have begun to define various stages in the complex cellular targeting mechanisms, the details of environmental adaptation or host-microbial cross-talk remain to be described.

Recent studies by the present inventors have shown that Nm⁷⁻⁹ and Hi^{10,11} share some distinct and other common mechanisms of targeting certain human cell surface receptors CEACAMs ¹².

Furthermore, the present inventors have recently identified that clinical isolates of *Moraxella catarrhalis* also target the human CEACAM molecules. In addition, it has now been found that a Moraxella outer membrane protein of high molecular weight binds to the receptor. Expression of CEACAMs in distinct tissues ¹² including respiratory epithelial cells has been demonstrated ²⁴. These observations imply that specific targeting of CEACAMs is of particular advantage to respiratory bacteria and may have arisen as a result of convergent evolution.

Amongst the adhesion factors elaborated by *Neisseria meningitidis* are pili (fimbriae)^{13,14,16} and the outer membrane opacity proteins, Opa and Opc ^{15,16}. Nm pili are long filamentous protein structures composed of multiple pilin subunits. They are generally regarded as the most important adhesins in capsulate bacteria^{13,14,16} due the fact that capsule partly or totally masks outer membrane ligands resulting in their reduced functional efficacy, whilst pili traverse the capsule and remain functional in fully capsulate bacteria. Opa are antigenically variable family of proteins and occur *in N. meningitidis* as well as *N*. *gonorrhoeae.* In meningococci, 3 - 4 opa gene loci code for related transmembrane proteins with four surface exposed loops, three of which undergo sequence variation ^{16,17}. Opc, another trans-membrane protein, is largely invariant ^{15,16}. Over the last 12 years, the present inventors have investigated structure/function relationships of Nm pili, the virulence potential of Opa and Opc proteins and identified two human receptors for the neisserial opacity proteins. Further, the role of surface sialic acids in bacterial interactions with human target cells as well as the role of LPS and other factors in cellular toxicity have been studied by the present inventors.

The present disclosure refers to the identification by the inventors of a ligand of high molecular weight isolated from Moraxella outer membrane protein which binds to CEACAM receptors.

The ligand can be characterised by its SDS-PAGE migration pattern which is indicative of the USP-family of proteins in that it is broken down to monomers having a molecular weight of between approximately 60 and 150 kD when boiled for a prolonged period.

The ligand has been characterised in Mx strain ATCC 25238 (MX2) as UspA1 and its amino acid sequence determined. The ligand has been further characterised to determine the receptor binding region or domain, i.e. peptide or peptide-associated features that bind to the receptor.

Accordingly, the present invention provides a ligand isolated from *Moraxella catarrhalis* outer membrane protein which binds to CEACAM receptors, said ligand being defined in the attached claims.

The term ligand is used herein to denote both the whole molecule which binds to the receptor and any part thereof which includes the receptor binding domain such that it retains the receptor binding property. Thus "ligand" encompasses molecules which consist only of the receptor binding domain *i.e*. the peptide region or regions required for receptor binding

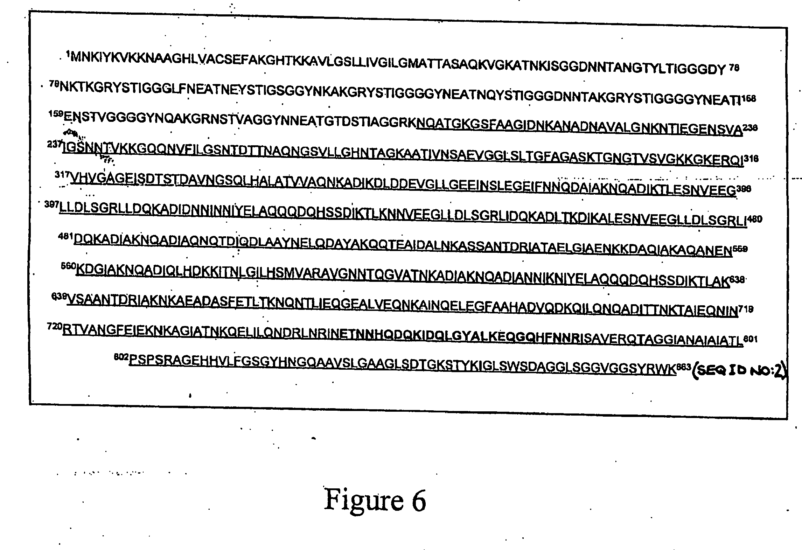

In one embodiment, the present disclosure refers to a ligand consisting of an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.

Structurally and/or functionally equivalent receptor binding domains may also occur in other UspA-like proteins since hybrid proteins occur in Mx that may contain mosaic epitopes derived from both UspA1 and UspA2 proteins ²³. Ligands comprising such equivalent receptor binding domains are also within the scope of the invention.

Preferably the ligand or receptor binding domain is suitable for use in the prevention or treatment of infection.

The present disclosure also concerns a nucleic acid sequence encoding the ligand protein of the present invention together with homologues, polymorphisms, degenerates and splice variants thereof.

The ligand of the invention, or combinations thereof may be used in a vaccine or other prophylactic treatment of infection.

The vaccine or other prophylactic treatment may comprise any known adjuvant, vehicle, excipient, binder, carrier, preservatives and the like, to provide a pharmaceutically acceptable preparation of the ligand for use in the treatment of a patient.

The invention also provides a pharmaceutically acceptable preparation of the ligand for use in medicine.

The pharmaceutically acceptable preparation of the ligand may be used in the treatment or prevention of any disease where CEACAM receptors are implicated, for example in the treatment or prevention of infection, respiratory disease, neoplastic diseases and associated conditions of neoplastic diseases, and angiogenesis.

Preferably, where an infection is treated, the infection is of, or has occurred via, the mucosal membrane, especially a respiratory infection.

Most preferably, the ligand is used as a vaccine for or in other prophylaxis or treatment of *Neisseria meningitidis, Haemophilus influenzae* and *Moraxella catarrhalis.* Ideally, the ligand is used as a vaccine for or in other prophylaxis or treatment of otitis media.

In a further aspect, the ligand of the present invention may also be used to identify novel blocking reagents for use as therapeutic agents to protect vulnerable groups and the public in general against several mucosal pathogens. For example the ligand may be used to identify receptor analogs which are useful for this purpose.

Hence the present disclosure also provides a screening assay for the identification of novel blocking reagents for use as therapeutic agents, the assay comprising the steps of screening potential therapeutic agents for their ability to mimic or for their homology to the ligand of the present invention. The invention further provides therapeutic agents identified by the aforementioned screening assay.

Effective vaccine components may be produced by using the information of the receptor targeting mechanisms identified by the present invention such as biologically active peptide mimics. These could prevent bacterial colonisation / invasion of mucosa as well as elicit antibodies which may be blocking, opsonic and bactericidal.

Bacterially derived biologically active peptide sequences identified by the ligand of the present invention could be used to study the roles of CEACAMs in cancer and development as the molecules are associated with these processes. These also have potential as anti-cancer agents and to control or otherwise in the treatment of angiogenesis.

In a further embodiment, the invention provides the use of a CEACAM receptor-binding ligand in the manufacture of a medicament for the treatment or prophylaxis of a disease in which CEACAM receptors are involved in cellular targeting of the pathogen which causes the disease according to appended claim 12.

Preferably, the disease is selected from the group consisting of infection, respiratory disease, neoplastic disease and associated conditions of neoplastic disease, and angiogenesis.

Medicaments as described above are of particular utility where the pathogen infects, or enters via, a mucosal membrane.

Medicaments as described herein are particularly useful in the treatment or prevention of infections (disease) caused by *Moraxella catarrhalis.* However, ligands as described herein are useful in the manufacture of medicaments for the treatment of any disease where CEACAM receptors are implicated such as diseases caused by *Neisseria meningitidis* and

*Haemophilus influenzae.*

In a particularly preferred embodiment, the disease is otitis media.

Ligands of the invention may also be used in the treatment of diseases caused by other oral bacteria, such as dental caries.

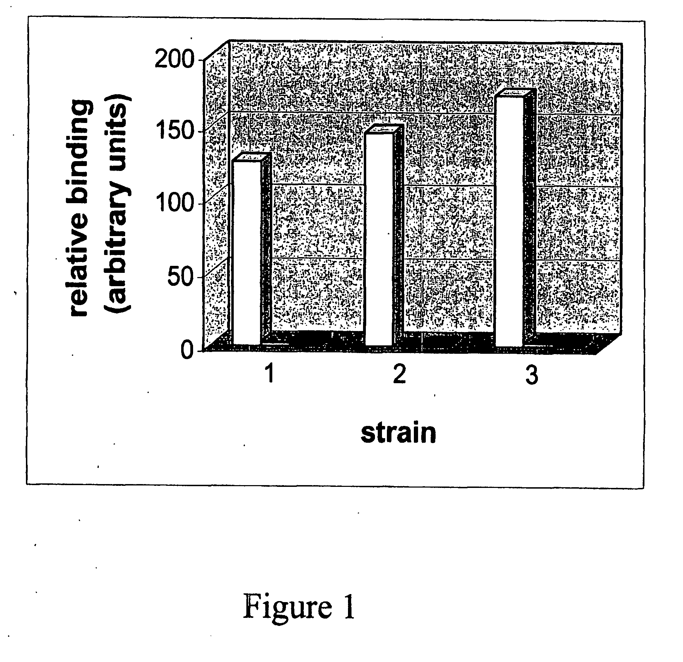

Embodiments of the present invention will now be described purely by way of non-limiting example in which reference is made to the figures of which:-
Figure 1 is a graph showing relative binding levels of CEACAM1-Fc (1µg.ml⁻¹) soluble receptor construct alone (white, left hand bars), or in the presence of the CEACAM1 N-domain specific antibody YTH71.3 (grey, centre bars) to 3 strains of Mx immobilised on nitrocellulose. CD33-Fc binding in each case was negligible (black, right hand bars). Strains 1, 2, 3: MX2 (ATCC 25238), MX3, MX4 (clinical isolates) respectively. Binding was determined in a dot blot overlay and the intensity of reactions quantified by densitometric analysis using NIH Scion Image program.

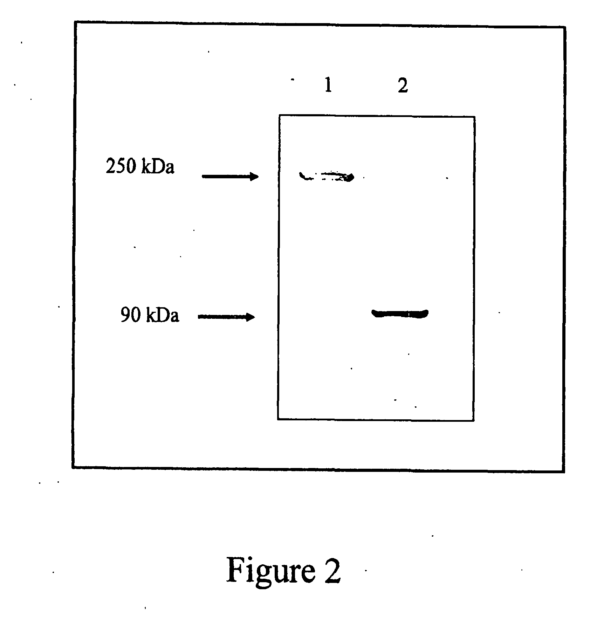

Figure 2 shows a Western blot of the strain MX2 proteins separated under undissociating (unheated, lane 1) or after boiling for 10 min. (lane 2). Blot was overlaid with CEACAM1-Fc (1 µg.ml⁻¹) and the receptor binding detected with anti-human Fc antibody conjugated to horseradish peroxidase and its substrate.

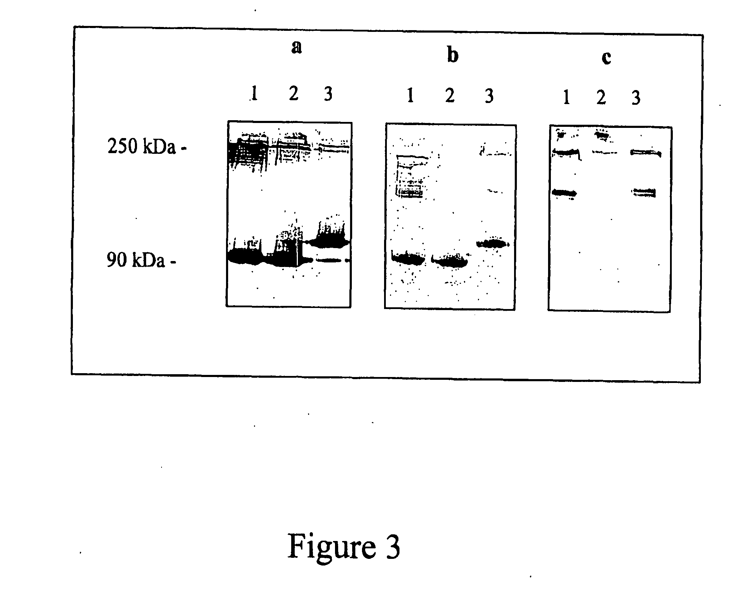

Figure 3 shows Western blots of denatured whole cell lysates of strains MX2, -3, -4 (lanes 1-3 respectively) were overlaid with CEACAM1-Fc (1 µg. ml⁻¹; a), anti-UspA1 peptide antibody (10 µg.ml⁻¹; b) and anti-UspA2 peptide antibody (10 µg.ml⁻¹; c). Note the similar migration profile of CEACAM1-Fc binding proteins and anti-UspA1 binding proteins in the three strains. Remnants of undissociated proteins at c. 250 kDa, (detected in this case due to higher sensitivity of detection in the alkaline phosphatase assay), bind to CEACAM1-Fc. These are only weakly recognized by the anti-peptide antibodies, presumably since the epitopes contained within synthetic peptides are not fully exposed in the native protein, which become progressively exposed as the complex denatures. Anti-UspA2 antibodies bind to proteins of apparent masses > 200 kDa, which remain undissociated after boiling, a property indicative of UspA2 proteins.

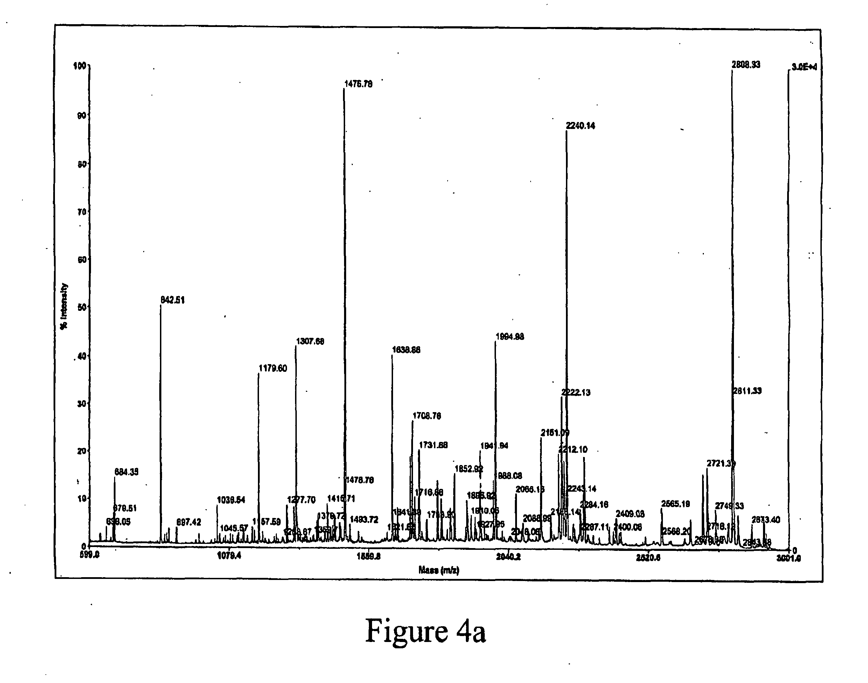

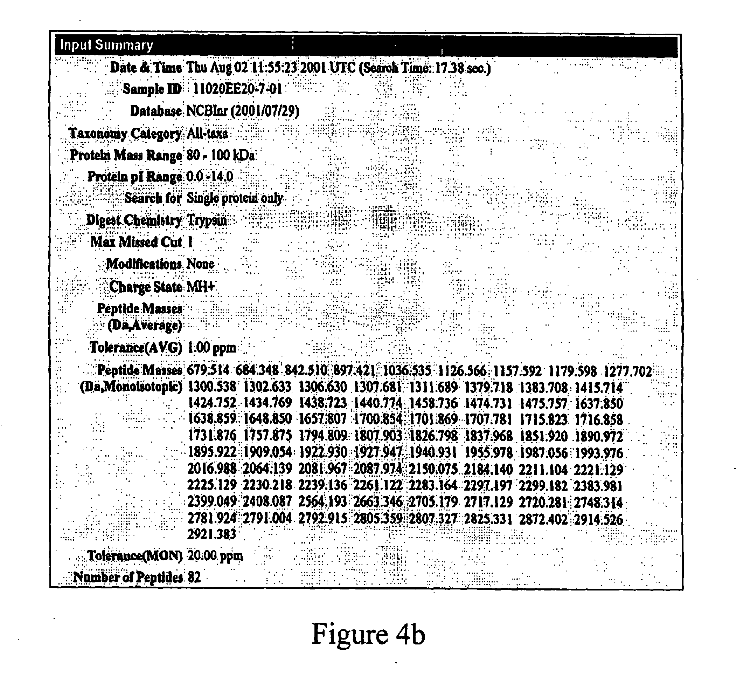

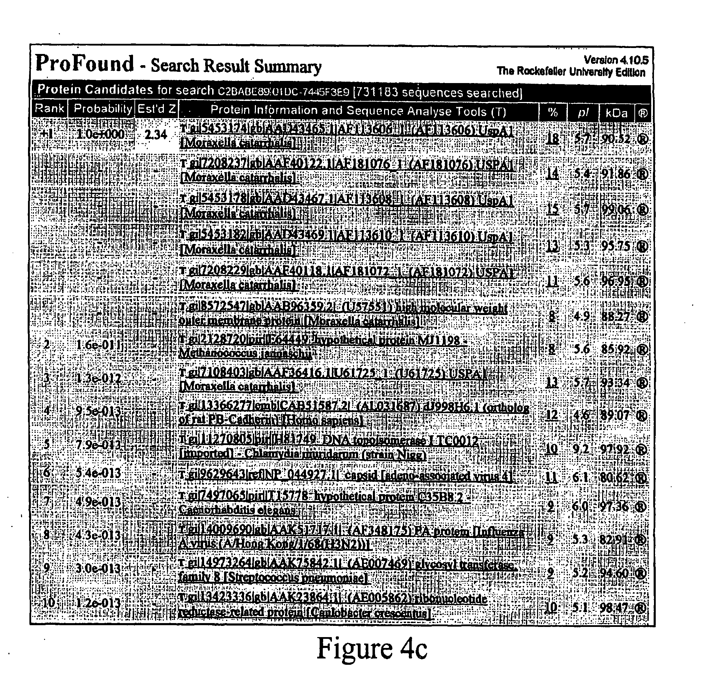

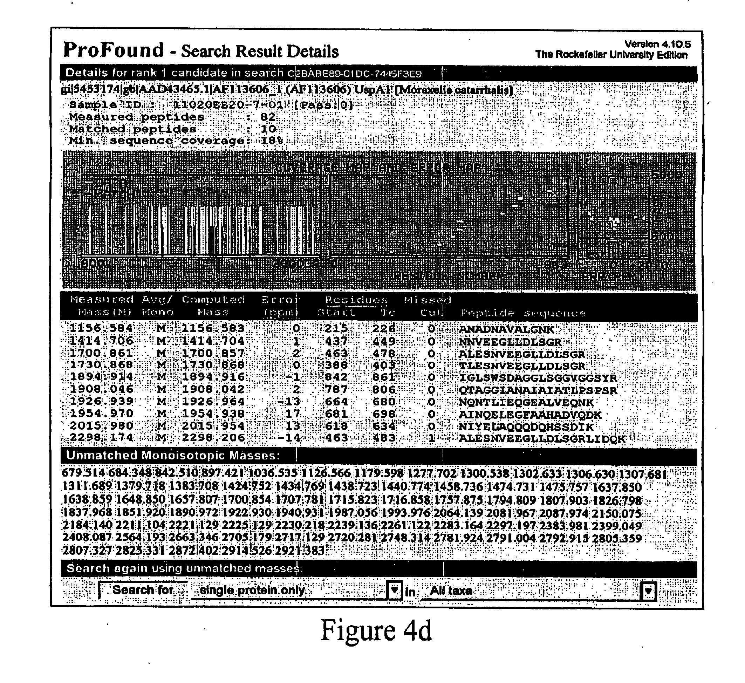

Figure 4 shows a mass spectrum of tryptic peptides of the CEACAM1 binding protein of MX2 following electro-elution (4a). The summery of data input into the ProFound protein identification database is shown in (4b). A table of the top ten identified proteins and the probability values are shown in (4c). Detail of the number 1 ranked candidate, in this case UspA1 with a Z-score of 2.34 is shown in (4d) indicating the number of peptides matched, their positions within the protein, the % of the protein covered and a list of peptides unmatched on this occasion.

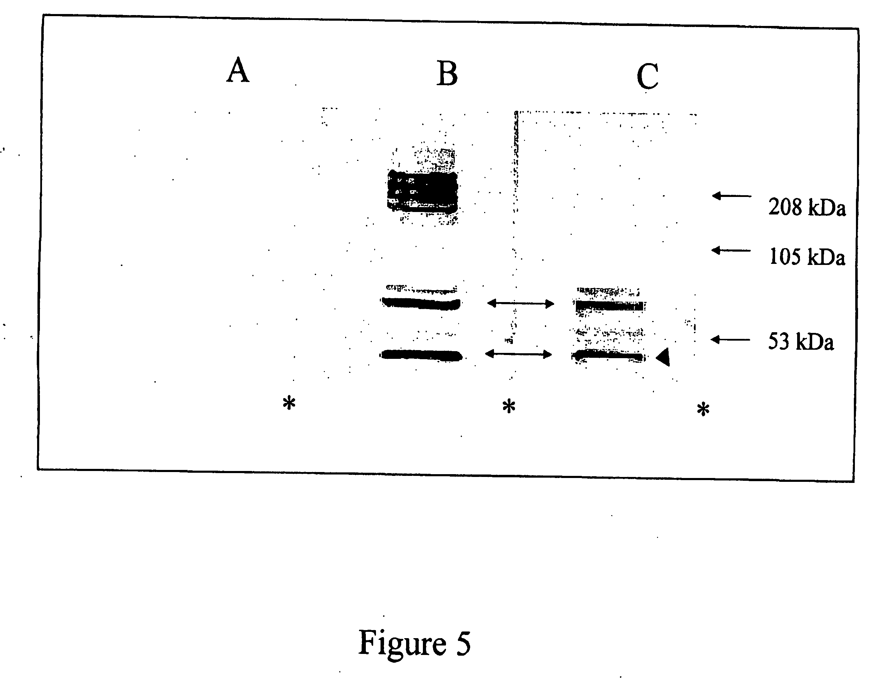

Figure 5 shows Western blot analysis of tryptic fragments of UspA1 of MX2. A: control blot using secondary antibodies (mixture of goat anti-human Fc and goat anti-rabbit Ig used in B and C). B: blot overlaid with CEACAM1 - Fc and goat anti-human Fc. C: blot overlaid witch affinity purified rabbit antibodies raised against UspA1 peptide (ETNNHQDQKIDQLGYACKEQGQHFNNR (SEQ ID NO:1)) (see Figure 6) and anti-rabbit Ig. * = lanes with molecular weight markers - shown on the left. The peptides shown by the double arrows react strongly with CEACAM1-Fc (B) as well.as the anti-UspA1 peptide antibodies (C). The binding of the anti-UspA1 peptide antibodies to the lowest MW peptide (arrowhead) identifies this CEACAM-binding fragment as the C-terminal fragment contained within N-199 to K-863 of UspA1 of MX2 (see Figure 6).
Figure 6 shows the amino acid sequence of MX2 UspA1 protein. UspA1-specific peptide used to raise antisera in rabbits is shown in bold. The CEACAM-binding region is contained in the underlined C-terminal fragment of UspA1 of MX2.

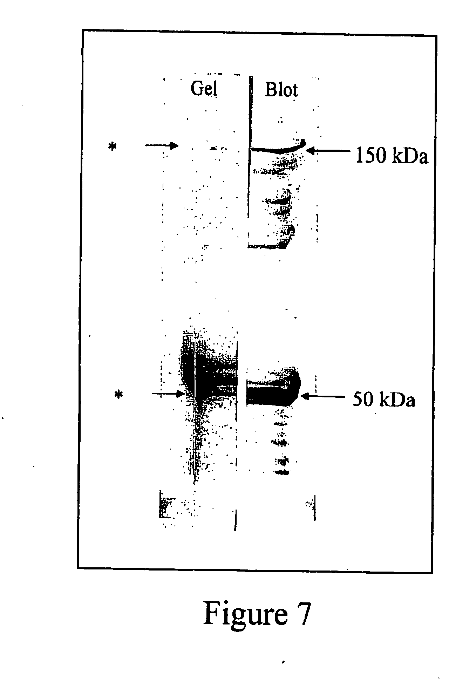

Figure 7 shows the separation of tryptic peptides reacting with CEACAMs. *M. catarrhalis* strain MX2 was treated with 1mg/ml trypsin at 37°C for 10 min. Trypsinised sample was subjected to SDS-PAGE. After staining, 50 kDa region was electroeluted overnight. Electroeluted protein was freeze dried and resuspended in buffer and applied to a second gel. Part of the gel was blotted onto nitrocellulose and peptide bands reacting with CEACAM were identified by Western blot overlay using CEACAM1-Fc (Blot).
   '*': denotes peptide bands sent for N-terminal sequencing.

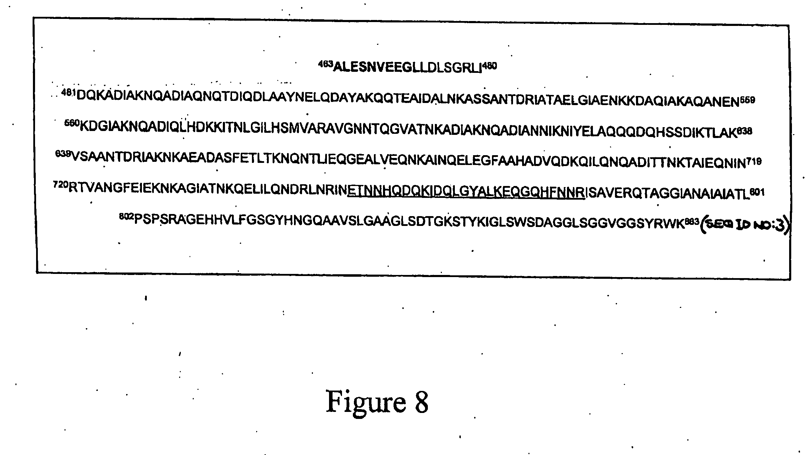

Figure 8 shows the amino acid sequence of a tryptic peptide of MX2 UspA1 protein. The 50 kDa tryptic peptide shown (amino acids 463 to 863) binds CEACAM and antiserum against UspA1 peptide (amino acids 753-780 underlined). The N-terminal sequence of the *c*. 50 kDa CEACAM binding peptide is "ALESNVEEGL" (SEQ ID NO:4) that occurs after the trypsin cleavage site at amino acid 462.

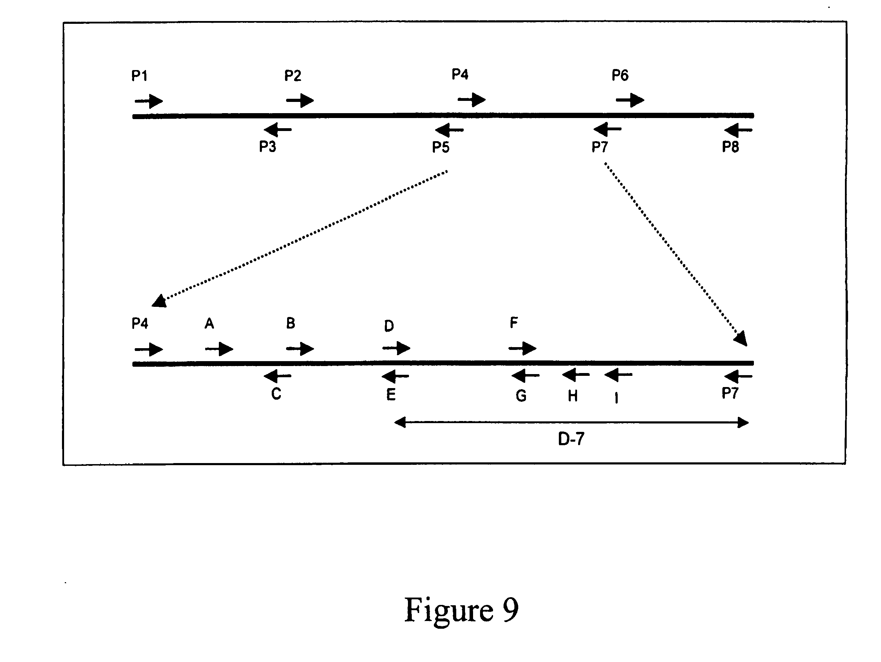

Figure 9 shows a diagrammatic representation of the positions of primers used to generate *uspA1* gene fragments for the expression of recombinant peptides. The CEACAM1 binding site was encoded by DNA amplified by primers P4 and P7, additional primers throughout this region (letters A-I) were designed and employed.

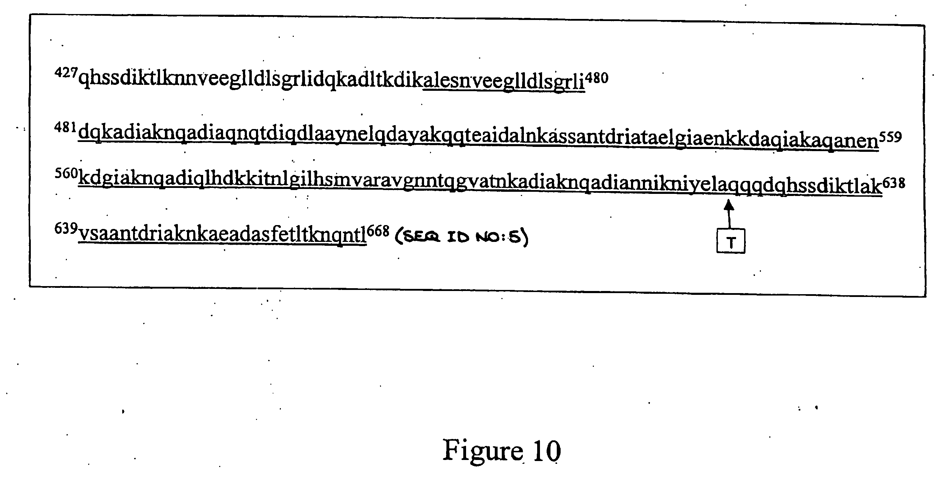

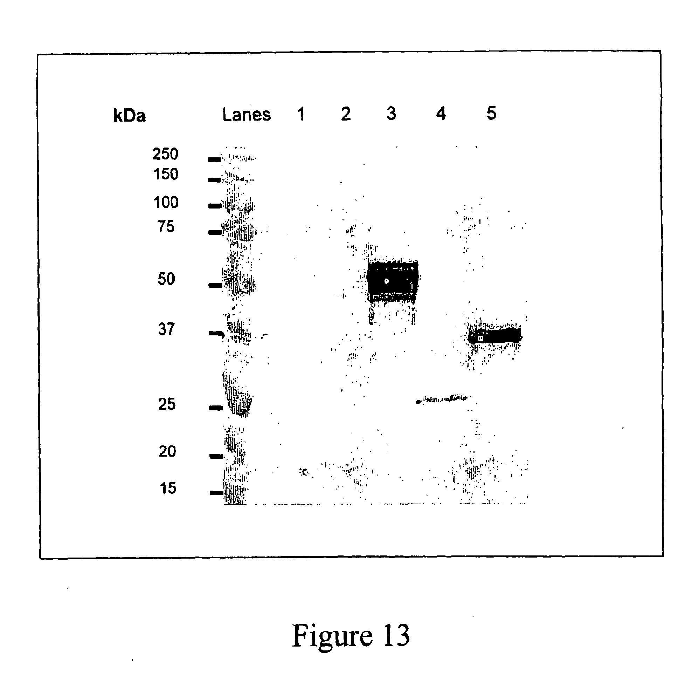

Figure 10 shows the sequence of the recombinant fragment 4-7. The underlined region is the N-terminal region of the CEACAM1-reactive tryptic peptide. The predicted molecular weight of the fragment 4-7 is c. 26 kDa. The predicted MW of the His-tagged fragment c. 28kDa. The position of the truncated peptide is shown by 'T' (see Figure 13).

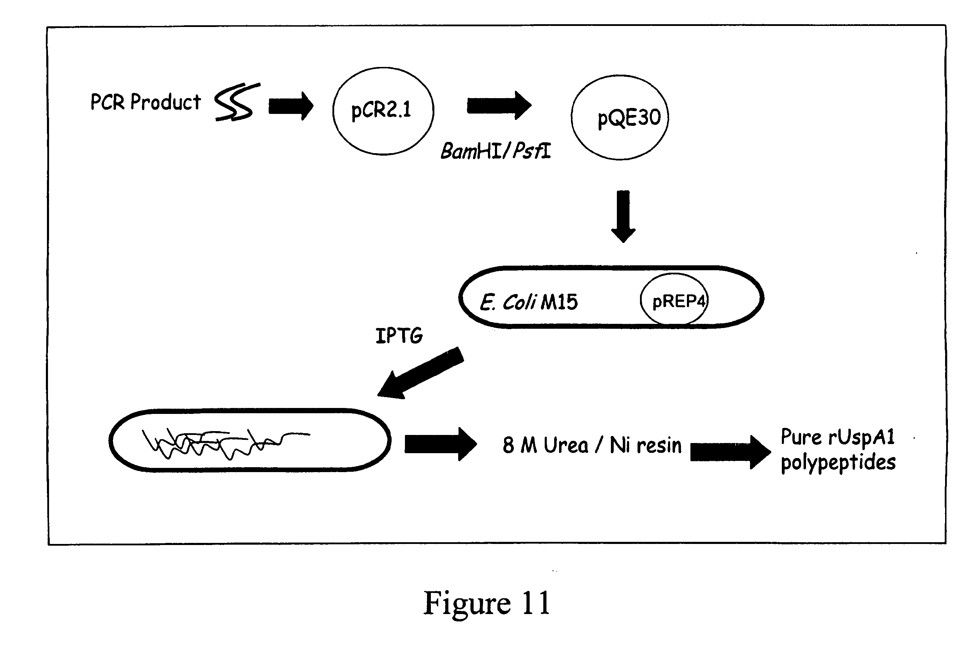

Figure 11 is a diagram showing the general cloning, expression and purification strategy for recombinant UspA1 peptides, as exemplified by the pQE30 system.

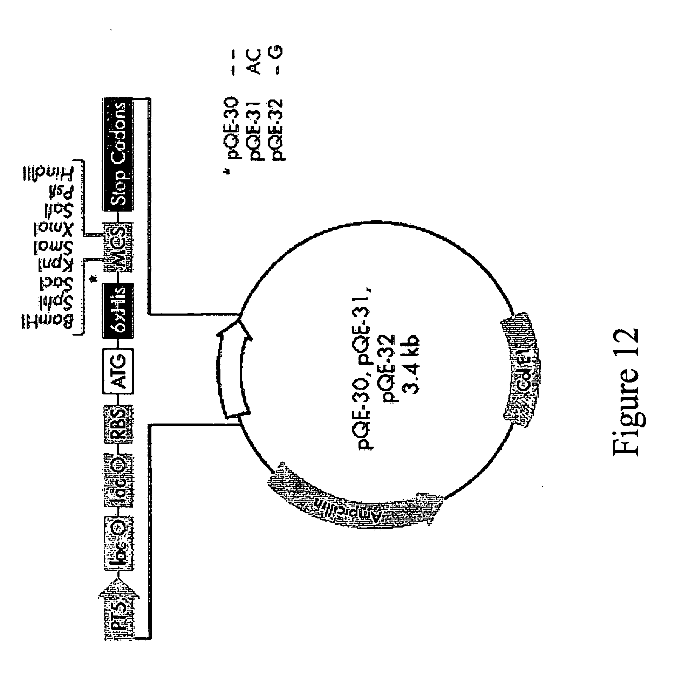

Figure 12 is a map of the vector pQE30.
Figure 13 shows binding of CEACAM1-Fc in blot overlay to recombinant 4-8 (lane 3), 4-T (lane 4) and 4-7 (lane 5) polypeptides. Lane 1 contained a Treponemal control recombinant peptide and lane 2 contained lysates of non-induced M15 containing 4-8 construct.

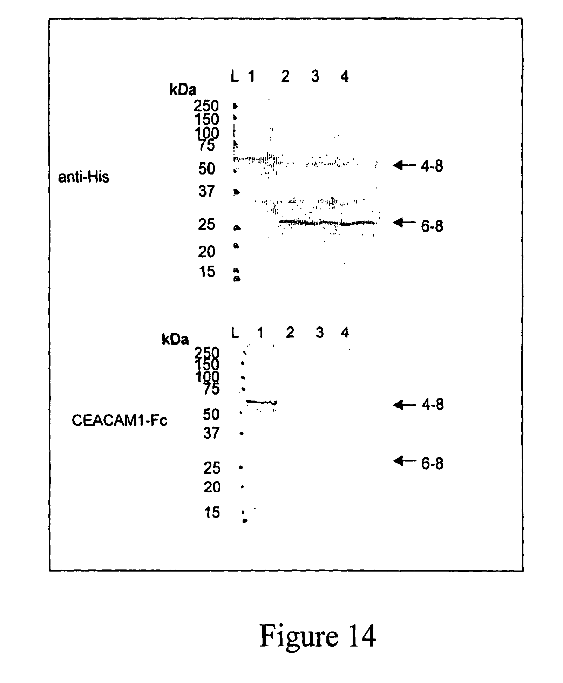

Figure 14 shows Western blots showing recombinant peptide reactivity with anti-His tag antibody (top) and CEACAM1-Fc (bottom). Lanes 2-4 contained 6-8 peptide, lane 1 contained 4-8 as a control. Predicted migration positions of the peptides are shown on the right. Both peptides bind anti-His antibody. However, whilst 4-8 binds to CEACAM1-Fc, 6-8 does not.

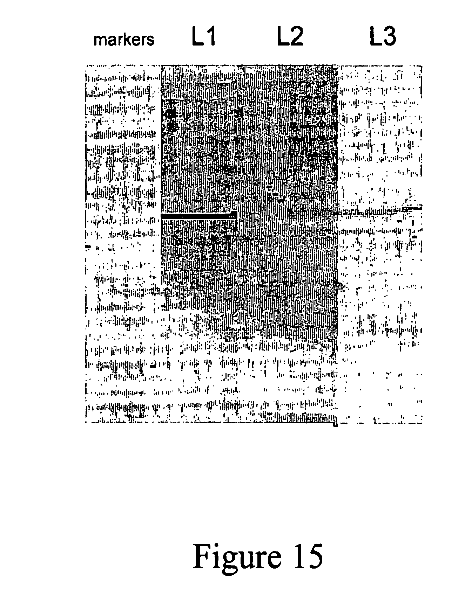

Figure 15 shows that D-8 polypeptide binds CEACAM1-Fc (lane 1) but not CD33-Fc used as a control (lane 2). The origin of this peptide was confirmed by reactivity with the anti-His tag antibody (lane 3).

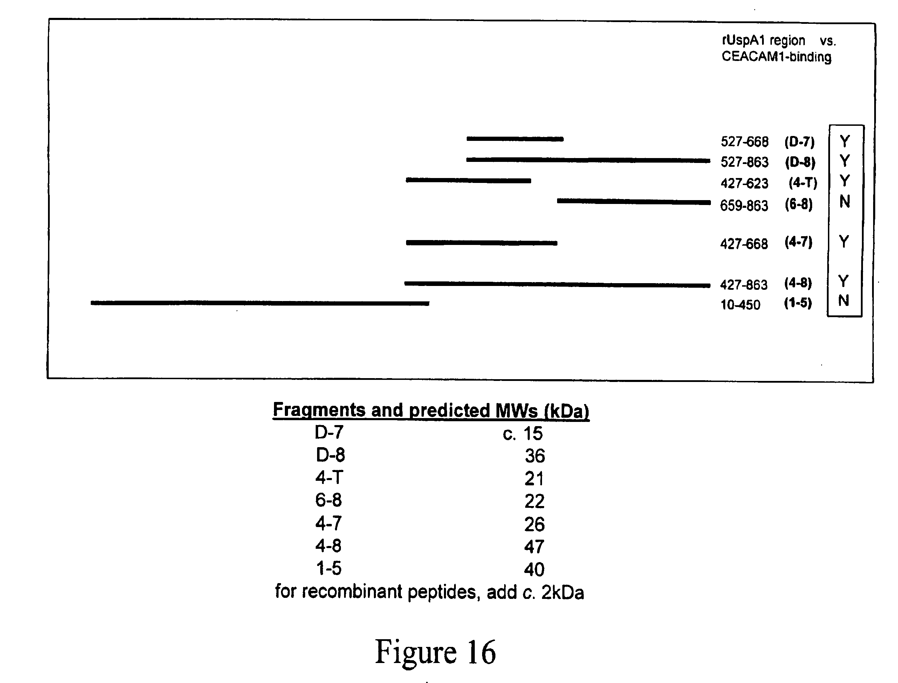

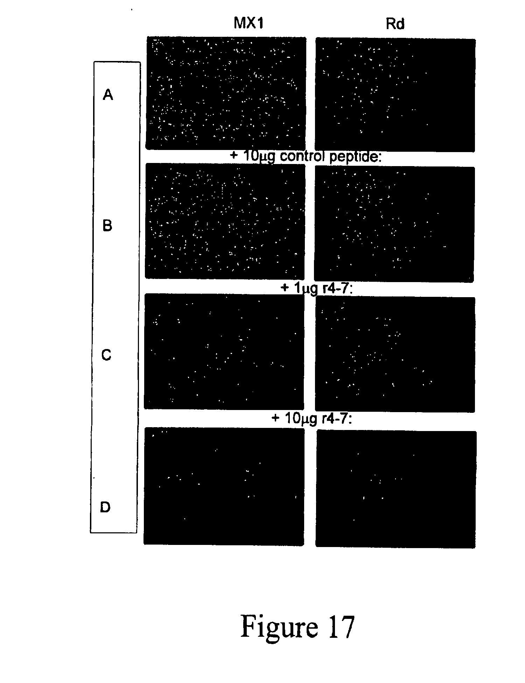

Figure 16 is a schematic diagram showing the relative sizes and positions of rUspA1 fragments. Recombinant 4-7 has been used for bacterial blocking - see Figure 17.
Figure 17 shows CHO-CEACAM1 transfectants were incubated in the absence of peptides (A) or with recombinant control peptide (a treponemal peptide, B) or UspA1 r4-7 peptide (sequence corresponding to the strain MX2, C and D) at the concentrations shown and bacteria added for a period of 2 hours. At the end of this incubation, unbound bacteria were washed off and bound bacteria detected with anti- *M. catarrhalis* polyclonal antiserum and TRITC conjugated secondary antibody. At 1 µg/ml significant inhibition of a heterologous *M*. *catarrhalis* strain (MX1) was obtained and at 10 µg per ml, *H*. *influenzae* binding was significantly inhibited by *M*. *catarrhalis* UspA1 recombinant peptide.

### Example 1. Moraxella catarrhalis strains bind to human CEACAM1-Fc via the UspA1 proteins

The strains of *Moraxella catarrhalis* (Mx) used in this study include clinical isolates (MX3 and MX4) as well as reference strain purchased from American Type Culture Collection (ATCC 25238, a clonal culture of this was designated MX2).

### Receptor overlay assays show that Mx bind to CEACAM1-Fc receptor constructs

To assess the interaction of *M. catarrhalis* strains with CEACAM1, bacteria (c. 4 - 8 x 10⁶) were applied to nitrocellulose, air dried and nonspecific binding sites blocked in 3 % BSA-PBST. Nitrocellulose strips were overlaid with either CEACAM1-Fc (1-2 µg ml⁻¹) alone or in the presence of the CEACAM1 N-domain antibody YTH71.3. CD33-Fc (1-2 µg ml⁻¹) was used as a negative control. Chimeric protein constructs were prepared as previously described¹¹. Chimeric receptor binding was detected by goat anti-human Fc antibodies conjugated to either horseradish peroxidase or alkaline phosphatase. Blots were developed with either diamino benzidene and hydrogen peroxide or nitroblue tetrazolium and 5-bromo-4-chloro-3-indoylphosphate substrates respectively. All Mx strains bound to CEACAM1-Fc but not to CD33-Fc (Fig.1). The receptor binding was inhibited in the presence the monoclonal antibody YTH71.3 suggesting that the strains bound to the N terminal domain of the receptor. Accordingly, all the strains bound squally well to the truncated N-Fc construct of CEACAM1, containing only the N domain (not shown).

### Identification of CEACAM1-Fc binding protein of M. catarrhalis

Whole cell lysates of Mx (c. 3 x 10⁷) were applied to individual lanes of a 10% bis-tris polyacrylamide gel (Invitrogen) either without previous heat treatment or after heating at 100°C for 10 minutes and subjected to electrophoresis for 45 min at 180 V. Proteins from the gels were transferred to nitrocellulose membranes using standard blotting conditions. Nitrocellulose membranes were overlaid with soluble chimeric constructs as described above. In each case, a single protein was observed that bound specifically to CEACAM1-Fc. The migration of the protein on gels was indicative of UspA1 protein of Mx, since when bacterial lysates were applied without heat denaturation, the CEACAM1 binding proteins migrated with an apparent mass of > 200 kDa whereas when the lysates were first heated, the CEACAM1-binding proteins migrated with a reduced mass of *circa* 92 kDa (Fig. 2)

### Antibodies raised against UspA1 but not those against a similar protein UspA2 bind to the CEACAM1-Fc binding proteins of Mx strains

Peptides were designed according to published sequences for the UspA proteins of *M*. *catarrhalis* strains. Namely, ETNNRQDQKIDQLGYALKEQGQ HFNNR (SEQ ID NO:1; UspA1-peptide) and KDEHDKLITANKTAIDANKAS (SEQ ID NO:6; UspA2-peptide). Peptides were coupled to KLH via an N-terminal cysteine residue that was incorporated and were used to immunise rabbits (200 µg peptide per rabbit) at 14 day intervals, initially with complete Freund's adjuvant and thereafter with incomplete Freund's adjuvant. Rabbits were bled at day 0 and at 14 day-intervals post immunisation. Polyclonal antibodies were purified using the appropriate peptide coupled to AminoLink Plus columns (Pierce). UspA-specific antibodies were used at a concentration of 1-10 µg ml⁻¹ for Western blot overlays and detected with goat anti-rabbit secondary antibody coupled to alkaline phosphatase. Blots were developed as described earlier. The migration of the CEACAM1-Fc binding proteins on SDS-PAGE of the three Mx strains was identical to that of the UspA1-antibody binding proteins but not UspA2-antibody binding proteins (Fig. 3)

### M. catarrhalis ligand co-precipitating with CEACAM1-Fc is identified as UspA1

Overnight cultures of bacteria were suspended in 100 mM octyl βD glucopyranoside in PBSB containing a protease inhibitor cocktail (pic; PMSF 1 mM, E-64 1 µM, pepstatin A 1 µM, bestatin 6 nM and EDTA 100 µM). Samples were mixed end over end overnight at 4°C. Meanwhile 100 µl protein A coupled to sepharose CL-4B (Sigma) was incubated with either, 20 µg CEACAM1-Fc or CD33-Fc (used as a control) overnight at 4°C and subsequently washed 3 times with PBSB to remove any unbound receptor. Insoluble bacterial material was removed by centrifugation at 15, 000 g for 30 min. Soluble extract was incubated with either receptor-Protein A sepharose complex for 2h at 4°C (at a ratio of 5 x10⁸ bacteria per µg of receptor construct). Following extensive washing with 50 mM octyl βD glucopyranoside and PBSB samples were analysed by SDS-PAGE electrophoresis and Western Blotting under denaturing conditions.

In co-precipitation experiments with CEACAM1-Fc, MX4 yielded a strongly staining protein of c. 97 kDa and MX3 yielded a relatively weakly staining protein of c. 92 kDa. The masses of co-precipitated proteins corresponded to those observed in the receptor overlay experiments (shown in Fig. 3). Neither protein co-precipitated with CD33-Fc. The co-precipitated proteins were further identified as UspA1 proteins since they bound to anti-UspA1 peptide antibody. In addition, following excision from the gel, the MX4 protein was subjected to MALDI-TOF mass spectrometry (see below).

### CEACAM1 ligand identification by MALDI-TOF mass spectrometry

(a) Western overlay samples. Whole cell lysates of MX2 and MX3 were subjected to SDS-PAGE in trench gels and the protein band corresponding to CEACAM1-Fc binding ligand was electroeluted from the gel. The sample was concentrated and reapplied in a single lane of a second gel, subjected to electrophoresis prior to in-gel trypsin digestion of the appropriate protein. The resulting peptides were analysed by Matrix-assisted Laser desorption/ionisation-time-of-flight (MALDI-TOF) mass spectrometry. An example of the resulting mass spectrum is shown for the CEACAM binding protein of MX2 (Fig 4a). The peptide masses obtained were entered into the ProFound protein identification site and the results were obtained as shown for this protein (Fig. 4b, c). In this case 10 peptide masses were matched to predicted masses for tryptic peptides of UspA1 of *M. catarrhalis* covering approximately 18 % of the protein (Fig. 4d). The estimated Z score of 2.34 is strongly suggestive of the protein being UspA1 (Z score >1.65 are above the 95^{th} percentile; http:/129.85.19.192/profound_bin/webProFound.exe). In addition, another non-binding high molecular weight band of MX2 was identified as UspA2 following a similar analysis. Similarly for strain MX3, the CEACAM1 binding and non-binding proteins were identified as UspA1 and UspA2 respectively.
(b) Co-precipitated samples: For MX4, the CEACAM1-Fc co-precipitated protein (as described above) was also identified as UspA1 by MALDI-TOF MS with a Z score of 2.27 in an all taxa search. 12 peptides were matched covering 21 % of the protein.

This study therefore has identified that *Moraxella catarrhalis* targets human CEACAM1 via the high molecular weight protein UspA1 in the reference and clinical strains indicated.

### Enzymatic cleavage of UspA1 and recombinant peptide

### :(a) Tryptic peptides of UspA1 bind to CEACAM1-Fc

MX2 bacterial suspensions (10¹⁰ ml⁻¹) were treated with trypsin (Sigma) at 0.1-1 mg/ml concentrations and incubated for 1-4 hours at 37°C. Digested lysates were dissociated in SDS-PAGE buffer, boiled and subjected to electrophoresis. After transfer to nitrocellulose, the blots were overlaid with the receptor construct CEACAM1-Fc or the affinity purified anti-UspA1 peptide antibodies. A small fragment reacting with the receptor also bound to the anti-UspA1-specific antibodies (Fig. 5).

### (b) Localisation of CEACAM binding domain of UspA2 of M. catarrhalis strain MX2

MX2 bacterial suspensions were treated with Trypsin at 1mg/ml for 10 minutes at 37°C.

The trypsinised sample was run on an SDS-PAGE gel. After staining, the 50 kDa region was electroeluted overnight. Electroeluted protein was freeze dried and resuspended in buffer and applied to a second gel. Part of the gel was blotted onto nitrocellulose and peptide bands reacting with CEACAM were identified by Western blotting overlay using CEACAM1-Fc. (Fig.7). Peptide bands corresponding to circa. 50 kDa and circa. 150 kDa peptides were subjected to N-terminal sequencing. The N-terminal sequences were ALESNVEEGL (SEQ ID NO:4) (c. 50 kDa peptide) and ALESNV (SEQ ID NO:7) (c. 150 kDa peptide). The 150 kDa protein is apparently a trimer of the 50 kDa protein as they have the same N-terminal sequence. The N-terminal sequence of this peptide of MX2 UspA1 is shown in Figure 8.

### (c) Recombinant peptide

The N-terminal recombinant MX2 peptide that was constructed consisting of amino acids 1 to 449 of the sequence shown in Figure 6 does not bind CEACAM. This further indicates that the c. 50 kDa tryptic peptide consisting of amino acids 463 to 863 of the sequence shown in Figure 8, having the N-terminal sequence ALESNVEEGL (SEQ ID NO:4) contains the CEACAM-binding domain.

### Example 2. Identification of Receptor Binding Domains on Multiple Virulence Determinants of Mucosal Pathogens

*N. meningitidis and H. influenzae* target human CEACAM molecules via ligands that bind to overlapping sites on CEACAM. The Mx ligand/s of the present invention also target the N domain. These observations point to an exciting possibility that similar features on ligands of several mucosal pathogens may be involved in receptor targeting.

Since Nm and Hi interactions with CEACAMs are affected by the structural features of the surface expressed variable domains, this suggests that they may contain similar crucial amino acids in a spatial configuration that are able to bind to the receptor and these may be conserved in otherwise variable proteins. Indeed our studies and those of others suggest that the two hyper-variable loops of Opa (HV1 arid HV2) may be involved in CEACAM targeting ^{9.18}. One powerful technique that can identify possible determinants of interactions between ligands and receptors is phage display, which can be used to identify mimotopes (random sequences to mimic the binding domains)¹⁹ as well as aptamers (sequences more closely related to the original structure that inhibit ligand binding)²⁰.

The current invention also makes it highly feasible that the Mx ligand domain that binds CEACAMs will act as a mimic for other CEACAM-blinding mucosal pathogens and the structural features of this ligand will help identify the salient features required in Nm and Hi ligands for CEACAM N-domain targeting Antibodies to the Mx domain could have the potential of identifying other ligands that have the capacity to target the same, similar or closely positioned region of the receptor.

Identification of the minimal CEACAM1 binding domain of MX2 UspA1 is being undertaken using known methods of protein engineering and recombinant DNA technology. Recombinant peptides can be screened in vitro by receptor overlay assays described above to detect the domain of MX2 that binds to the receptor. His-Tagged peptides can be separated on a nickel column, His-Tag cleaved as required and used for immunising rabbits and mice to obtain antibodies for further investigations.

A peptide of suitable length for biological applications may be determined by examining immunological stimulatory properties as well other functions such as blocking of receptor binding.

### Example 3. Salient Features of the Receptor Required for Ligand Interactions

Since the N-domain of CEACAM is sufficient for the interactions of Opa proteins, the present inventors are using phage display technique also to investigate adhesive epitopes of the CEACAM N-domain. The knowledge of the ligand-binding region/s on the receptor, which has been studied by the present inventors by alanine scanning mutagenesis of the receptor⁹ has facilitated this study. In this case also, a ligand overlay assay is available for biopanning (affinity concentration) of chimeric phages that bear receptor sequences.

Receptor analogues have the potential to block multiple strains independently of the Opa type produced since our studies have already shown that despite their antigenic variation, distinct Opa proteins require common features on the receptor for primary adhesion⁹. It is also of interest to note that CEA antigens are shed from the gut mucosa and may block adhesion of *E*. *coli* strains also known to target CEACAMs. This has been proposed as a mechanism of innate immunity vs. enteric pathogens. ¹² Thus these receptor analogues act as therapeutic agents.

### Example 4. Production of Recombinant CEACAM-binding Moraxella catarrhatis UspA1 peptides

### Summary:

PCR amplification of several fragments along the length of UspA1 was carried out using the primers shown in Figure 9. Recombinant peptides were obtained as described below. In the first round, it was found that the recombinant peptide that bound to CEACAM1 Was encoded by DNA amplified by primers P4 and P8 but not that encoded by region between P1 and P5. Further, recombinant P4 - P7 bound CEACAM1 but not P6 - P8. Additional primers were used within P4 and P7 region. Region D-7 retained CEACAM-binding. The sequence of fragment 4-7 is shown in Figure 10.

### General cloning, expression and purification strategy for recombinant UspA1 peptides

The UspA1 fragment 1-5 was produced using the pBAD system. The required PCR products were TA cloned into pBAD vector and TOP10 *E*. *coli* strain was used for amplification. The rest of the procedure was as shown in Fig. 11 with the exception that pBAD was induced using arabinose. The fragment 4-7 was produced using both the pBAD and pQE30 (Fig. 11) systems with similar CEACAM-binding results. The rest of the fragments were produced using the strategy in Fig. 11.

### Vector and the pQE30 expression system

The vector pQE30 (Fig. 12) was used in conjunction with *E*. *coli* strain M15. M15 contains the plasmid pREP4 encoding a repressor, which restricts transcription of DNA cloned into pQE30. The addition of IPTG to a concentration of 1 mM prevents coding of this repressor and thus transcription of the cloned fragments in pQE30.

### The cloning strategy

PCR amplimers were first ligated into pCR2.1. This provided a stable host from which the amplimer could be recovered by restriction digest (*Bam*HI/*Pst*I), ensuring that each end of the gene fragment was cut. pQE30 was similarly digested and recovered by gel purification, which also ensured that both restriction sites had been cut. Digested pQE30 and UspA1 amplimer were ligated overnight at 16°C with T4 DNA ligase and transformed into CaCl₂ competent *E. coli* M15. Transformants were selected for on LB agar supplemented with ampicillin (100 µg/ml) and kanamycin (25 µg/ml). 4-8 colonies were picked and grown in LB broth with antibiotics. Bacteria from 3-4ml of culture were collected by centrifugation and subjected to the alkaline-lysis miniprep method. Purified vector was digested as above to check for *uspA1* insert. Bacteria containing pQE30 with a correct sized insert were grown in 50 ml. cultures and induced with IPTG (see below). Western blots were then used to screen for recombinant protein production. In addition, vectors were sequenced to determine that the insert was the correct region of DNA and to check for any sequence errors.

### Expression and purification

M15 containing pQE30/*uspA1* constructs were grown in LB (supplemented with 100 µg/ml Ampicillin and 25 µg/ml Kanamycin) broth with shaking to OD600 = 0.5 before the addition of IPTG to a concentration of 1 mM. Cultures were incubated for a further 3-4 hours and bacteria recovered by centrifugation. Bacterial pellets were solubilised in buffer B (8M urea, 50mM Tris, 10% ethanol, 2% Tween, 5mM imidazole, pH 7) for 1-3 hrs and membrane material removed by centrifugation at 20,000 g for 20 min. Supernatants were incubated with nickel resin for 1-2 hrs on a rotary mixer, and passed through a polypropylene column. The retained resin was washed with 5-10 ml buffer B and bound protein eluted with 0.5 ml elutions of buffer B supplemented with 100 mM imidazole. Eluted proteins were checked by SDS-PAGE before dialysis to remove urea and other salts.

### Recombinant fragments

### A: Fragments 1-5 and 4-8

These fragments produced by pBAD system showed that 1-5 did not bind CEACAM1 but 4-8 did.

### B: Fragments 4-8, 4-8T, 4-7 and 6-8 produced using pQE30 system.

Fragment 4-8 was the first rUspA1 fragment produced and was found to bind CEACAM1 with a high affinity in blot-overlay assays. In addition to the full-length 4-8 rUspA1 peptide, a smaller, truncated, protein was observed. This peptide (designated 4-T) also bound CEACAM1 and appeared to be expressed at lower levels than 4-8 (Fig. 13). Sequence analysis of pQE30/4-T found that a mismatch (CAA to TAA) led to a termination codon at residue Q624 (see Fig. 10).

Peptides 4-7 and 6-8 were produced in order to rule out the possibility that a second CEACAM binding site occurred in 6-8. As predicted from the 4-T peptide, 4-7 demonstrated CEACAM binding (Fig. 13) but 6-8 did not (Fig. 14).

### C: Fragments D-8 and D-7 produced by pQE30 system

Both rUspA1 fragments D-8 (Fig. 15) and D-7 (not shown) were found to bind CEACAM1.

### Biological activity of recombinant peptide 4-7

*M. catarrhalis* strain MX1 and *H*. *influenzae* strain Rd bind to Chinese Hamster Ovary (CHO) cells transfected with CEACAM1. Their binding can be blocked with *M*. *catarrhalis* UspA1 recombinant peptide 4-7 but not a control peptide (Fig. 17).

### References:

1. Cartwright, K. et al. 1995. In Meningococcal Disease. John Wiley & Sons.
2. van Alphen, L. & van Ham, S. M.. 1994. Rev Med Microbiol 5:245.
3. Foxwell, A. R. et al. 1998. Microbiol Mol Biol Rev 62:294.
4. van Alphen, L. et al. 1995. Am J Respir Crit Care Med 151:2094.
5. Karalus, R. et al. 2000. Microbes & Infection 2:5
6. Kraft, M. 2000. Clin Chest Med 21:301.
7. Virji, M. et al. 1996. Mol Microbiol 22:929.
8. Virji, M. et al. 1996. Mol Microbiol 22:941.
9. Virji, M. et al. 1999. Mol Microbiol 34:538.
10. Virji, M. et al. 2000. Mol Microbiol 36:784*.*
11. Hill, D. et al. 2001 Mol Microbiol 39: 850.
12. Hammarström, S. 1999 Cancer Biol 9:67.
13. Stephens, D. S. 1989. Clin Microbiol Rev 2:S104.
14. Virji, M. et al. 1991. Mol Microbiol 5:1831*.*
15. Achtman, M. 1995 Trends Microbiol 3:186*.*
16. Merz, A. J. & So, M. 2000 Annu Rev Cell Dev Biol 16:423.
17. Woods, J. P. & Cannon, J. G. 1990 Infect Immun 58:569*.*
18. Wang, L-F & Yu, M. 1996 In Methods Mol Biol 66:269 (Morris, G. E. ed). Humana Press.
19. Colman-Lerner. 2000. Trends Guide p. 56 (Wilson, E. ed. ),
20. Beauchemin, N., et al., 1999 Exp Cell Res 252: 243-249.
21. Boulton I.C. & Gray-Owen S.D. 2002 Nat Immunol 3(3):229-36.
22. Chen T. et al., 2001 J Leukoc Biol 70(2):335-40.
23. Lafontaine, E.R., et al., 2000 J Bacteriol 182: 1364-1373.
24. Virji, M. 2001 Trends in Microbiology, 9: 258-259.

## Claims

1. A ligand which is a part of the whole UspA1 molecule including the CEACAM receptor binding domain, said ligand consisting of an amino acid sequence of SEQ ID NO: 3 or a fragment thereof required for CEACAM receptor binding.

2. The ligand according to claim 1, wherein the ligand consists of an amino acid sequence of SEQ ID NO: 3.

3. A medicament comprising the ligand according to claim 1 or claim 2 and one or more pharmaceutically acceptable adjuvants, vehicles, excipients, binders, carriers, or preservatives.

4. The medicament according to claim 3 for the treatment or prophylaxis of infection.

5. The medicament according to claim 3 for the treatment or prevention of a disease in which CEACAM receptors are involved in the cellular targeting of the pathogen which causes the disease.

6. The medicament according to claim 4 or claim 5, where the infection is of, or has occurred via, a mucosal membrane.

7. The medicament according to claim 6, wherein the infection is caused by *Neisseria meningitidis, Haemophilus influenzae* or *Moraxella catarrhalis.*

8. The medicament according to claim 3 for the prophylaxis or treatment of otitis media.

9. Use of the ligand according to claim 1 in a screening assay for the identification of novel blocking reagents for use as therapeutic agents comprising screening potential therapeutic agents for their ability to mimic, or for their homology to, said ligand.

10. A vaccine comprising the ligand according to claim 1 and one or more pharmaceutically acceptable adjuvants, vehicles, excipients, binders, carriers, or preservatives.

11. A ligand according to claim 1 or claim 2 for use in the treatment or prevention of infection.

12. A CEACAM receptor binding ligand comprising the amino acid sequence SEQ ID NO: 3 or a fragment thereof which retains CEACAM receptor binding ability for use in the treatment or prophylaxis of a disease in which CEACAM receptors are involved in cellular targeting of the pathogen which causes the disease.

13. A CEACAM receptor binding ligand according to claim 12 for use according to claim 12, wherein the pathogen which causes the disease is selected from the group consisting of *Neisseria meningitidis, Haemophilus influenzae* and *Moraxella catarrhalis.*

14. A CEACAM receptor binding ligand according to claim 13 for use according to claim 13, wherein the disease is otitis media.

## Patentansprüche

1. Ligand, der ein Teil des gesamten UspA1-Moleküls ist, einschließlich der Bindungsdomäne für den CEACAM-Rezeptor, wobei genannter Ligand aus einer Aminosäuresequenz der SEQ.-ID-Nr. 3 oder einem Fragment davon, das für die Bindung an den CEACAM-Rezeptor erforderlich ist, besteht.

2. Ligand nach Anspruch 1, worin der Ligand aus einer Aminosäuresequenz der SEQ.-ID-Nr. 3 besteht.

3. Medikament, das den Liganden nach Anspruch 1 oder Anspruch 2 und ein/en oder mehrere pharmazeutisch verträgliche Adjuvanzien, Vehikel, Hilfsstoffe, Bindemittel, Träger oder Konservierungsmittel umfasst.

4. Medikament nach Anspruch 3 zur Behandlung oder Prophylaxe einer Infektion.

5. Medikament nach Anspruch 3 zur Behandlung oder Prävention einer Erkrankung, bei der CEACAM-Rezeptoren am zellulären Targeting des Pathogens, das die Erkrankung verursacht, beteiligt sind.

6. Medikament nach Anspruch 4 oder Anspruch 5, wobei die Infektion die einer Schleimhaut ist oder über eine Schleimhaut aufgetreten ist.

7. Medikament nach Anspruch 6, worin die Infektion durch *Neisseria meningitidis, Haemophilus influenzae* oder *Moraxella catarrhalis* verursacht wird.

8. Medikament nach Anspruch 3 zur Prophylaxe oder Behandlung von Mittelohrentzündung.

9. Verwendung des Liganden nach Anspruch 1 in einem Screening-Test für die Identifizierung von neuen Blockierungsreagenzien zur Verwendung als Therapeutika, die das Screening potenzieller Therapeutika auf ihre Fähigkeit zur Nachahmung des oder auf ihre Homologie zum genannten Liganden umfasst.

10. Impfstoff, der den Liganden nach Anspruch 1 und ein/en oder mehrere pharmazeutisch verträgliche Adjuvanzien, Vehikel, Hilfsstoffe, Bindemittel, Träger oder Konservierungsmittel umfasst.

11. Ligand nach Anspruch 1 oder Anspruch 2 zur Verwendung bei der Behandlung oder Prävention einer Infektion.

12. Bindungsligand für den CEACAM-Rezeptor, der die Aminosäuresequenz SEQ.-ID-Nr. 3 oder ein Fragment davon, das die Bindungsfähigkeit an den CEACAM-Rezeptor beibehält, umfasst, zur Verwendung bei der Behandlung oder Prophylaxe einer Erkrankung, bei der CEACAM-Rezeptoren am zellulären Targeting des Pathogens, das die Erkrankung verursacht, beteiligt sind.

13. Bindungsligand für den CEACAM-Rezeptor nach Anspruch 12 zur Verwendung nach Anspruch 12, worin das Pathogen, das die Erkrankung verursacht, aus der Gruppe ausgewählt ist, die aus *Neisseria meningitidis, Haemophilus influenzae* und *Moraxella catarrhalis* besteht.

14. Bindungsligand für den CEACAM-Rezeptor nach Anspruch 13 zur Verwendung nach Anspruch 13, worin die Erkrankung Mittelohrentzündung ist.

## Revendications

1. Ligand qui est une partie de la molécule UspA1 complète comprenant le domaine de liaison du récepteur CEACAM, ledit ligand consistant en une séquence d'acides aminés de SEQ ID n° 3 ou un fragment de celle-ci requis pour la liaison du récepteur CEACAM.

2. Ligand selon la revendication 1, dans lequel le ligand consiste en une séquence d'acides aminés de SEQ ID n° 3.

3. Médicament comprenant le ligand selon la revendication 1 ou la revendication 2 et un ou plusieurs adjuvants, véhicules, excipients, liants, supports, ou conservateurs pharmaceutiquement acceptables.

4. Médicament selon la revendication 3 pour le traitement ou la prophylaxie d'une infection.

5. Médicament selon la revendication 3 pour le traitement ou la prévention d'une maladie dans laquelle des récepteurs CEACAM sont impliqués dans le ciblage cellulaire du pathogène qui provoque la maladie.

6. Médicament selon la revendication 4 ou la revendication 5, dans lequel l'infection est une membrane des muqueuses, ou s'est produite par l'intermédiaire d'une membrane des muqueuses.

7. Médicament selon la revendication 6, dans lequel l'infection est provoquée par *Neisseria meningitidis, Haemophilus influenzae* ou *Moraxella catarrhalis.*

8. Médicament selon la revendication 3 pour la prophylaxie ou le traitement de l'otite moyenne.

9. Utilisation du ligand selon la revendication 1 dans un dosage de dépistage pour l'identification de nouveaux réactifs de blocage à utiliser comme agents thérapeutiques comprenant le dépistage de la capacité d'agents thérapeutiques potentiels à imiter ledit ligand, ou leur homologie audit ligand.

10. Vaccin comprenant le ligand selon la revendication 1 et un ou plusieurs adjuvants, véhicules, excipients, liants, supports, ou conservateurs pharmaceutiquement acceptables.

11. Ligand selon la revendication 1 ou la revendication 2 à utiliser dans le traitement ou la prévention d'une infection.

12. Ligand de liaison du récepteur CEACAM comprenant la séquence d'acides aminés SEQ ID n° 3 ou un fragment de celle-ci qui conserve la capacité de liaison du récepteur CEACAM à utiliser dans le traitement ou la prophylaxie d'une maladie dans laquelle les récepteurs CEACAM sont impliqués dans le ciblage cellulaire du pathogène qui provoque la maladie.

13. Ligand de liaison du récepteur CEACAM selon la revendication 12 à utiliser selon la revendication 12, dans lequel le pathogène qui provoque la maladie est sélectionné parmi le groupe constitué de *Neisseria meningitidis, Haemophilus influenzae* et *Moraxella catarrhalis.*

14. Ligand de liaison du récepteur CEACAM selon la revendication 13 à utiliser selon la revendication 13, dans lequel la maladie est l'otite moyenne.
